# EUROPEAN PATENT APPLICATION

(11) **EP 2 006 374 A2**
(43) Date of publication of application: **24.12.2008**
(21) Application number: 08012238.5
(22) Date of filing: 14.09.2005
(51) Int. Cl.: C12N 5/06

(54) **Method for the purification and amplification of tumoral stem cells**

(30) Priority: 15.09.2004 IT RM20040438
(62) Divisional of application: 05794564.4
(71) Applicant: Apogenix GmbH, 69120 Heidelberg (DE)
(72) Inventor: Stassi, Giorgio, 90133 Palermo (IT); Todaro, Matilde, 90133 Palermo (IT)
(74) Representative: Weiss, Wolfgang

(57) **Abstract**

The invention concerns a method for the purification and amplification in the undifferentiated state of tumoral stem cells from solid tumours which are most resistant to conventional therapies, aiming at devising new tumour markers and therapeutic targets both for early diagnosis and for targeted therapeutic strategies.

## Description

The present invention concerns a method for the purification and amplification of tumoral stem cells. In particular, the present invention relates to a method to purify and amplify in undifferentiated state cancer stem cells from solid tumours, preferably of epithelial source. Moreover, the invention also concerns a method for inducing cancer stem cell death.

It is well-known that tumours originate from a tumorigenic cancer cell that acquires the capacity for indefinite proliferation through genetic alterations or mutations. Many observations suggest that analogies between tumorigenic cells and normal stem cells may be appropriate, in fact the principles of normal stem cell biology can be applied to better understand how tumours develop (Morrison et al., 1997; Weissman, 2000). Both normal stem cells and tumorigenic cells have extensive proliferative potential and the ability to give rise to new (normal or abnormal) tissues. Both tumours and normal tissues are composed of heterogeneous combinations of cells, with different phenotypic characteristics and different proliferative potentials. In particular, tumorigenic cancer cells may give rise to phenotypically diverse progenies of cells, either endowed with an indefinite proliferative potential, or having a limited or no proliferative potential. This suggests that tumorigenic cancer cells undergo processes that are analogous to the self-renew and differentiation of normal stem cells.

Stem cells are defined as cells that have the ability to perpetuate themselves through self-renewal and to generate mature cells of a particular tissue through differentiation (Reya et al., 2001).

Identification and isolation of somatic tissue-specific stem cells have been accomplished with good results only in few instances, such as, for example, in the case of haematopoietic stem cells isolated from mice and humans (Osawa et al., 1996; Morrison et al., 1994; Baum et al., 1992; Spangrude et al., 1988).

Therefore, both normal stem cells and tumorigenic cells give rise to phenotypically heterogeneous cells that exhibit various degrees of differentiation. Thus, tumorigenic cells can be regarded as cancer stem cells that undergo an aberrant and poorly regulated process of organo-genesis analogous to that typical of normal stem cells.

At present, all of the phenotypically diverse cancer cells are treated as though they had an unlimited proliferative potential and could acquire the ability to metastasize. However, it has been recognized that a small number of disseminated cancer cells can be detected at distant sites from primary tumours in patients that have never manifested metastatic disease. One possible explanation is that immune surveillance is highly effective in killing disseminated cancer cells before they can form a new detectable tumour. Another possibility is that most cancer cells lose the ability to form a new tumour such that only the dissemination of rare cancer stem cells can lead to metastatic disease.

If tumour growth and metastasis were driven by such small population of cancer stem cells, this might explain the failure to develop therapies that are consistently able to eradicate solid tumours. In fact, currently available drugs can act on tumours and shrink tumoral metastases by killing mainly cells with limited proliferative potential. On the other hand, the cancer stem cells are less sensitive to these therapies, and thus they will remain viable after therapy and re-establish the tumour. By contrast, if therapies could be targeted against cancer stem cells, the drugs might more effectively kill this type of cancer stem cells, thus rendering the tumours unable to survive. Recent data showed that cancer cells autocrine Th2 cytokines, such as Interleukin-4 and Interleukin-10, that are responsible for up-regulation of antiapoptotic proteins as Bcl-2, Bcl-xl and FLIP, which protect the tumour cells from death by exposure to conventional drugs (Stassi et al 2003).

In view of the foregoing, an object of the present invention is to provide a method to purify and amplify in undifferentiated state cancer stem cells present in solid tumours, preferably of epithelial source, in order to identify potential diagnostic markers and therapeutic targets expressed by said cancer stem cells. Concerning the method for inducing cancer stem cell death, the invention refers to the use of Th2 cytokine neutralizing antibodies, such as interieukin-4 (IL-4) and interleukin-10 (IL-10).

To this aim, there has been set up a method to purify and amplify in undifferentiated state from epithelial tumours the cancer stem cells most resistant to conventional therapy (i.e. chemotherapy), the said method being intended to identify new tumour markers and therapeutic targets both for early diagnosis and for targeted therapeutic strategies.

Actually, as above described, cancer stem cells are responsible to induce a minimal residual cancer disease, considering that most of the cells making up tumours are constantly renewed by a small population (1 %) of cancer stem cells.

According to the present invention, a method was developed to purify and amplify cancer stem cells which are normally located inside tumour mass and that, until today, it was impossible to characterize. This innovative technique permits to characterize and spread cancer stem cells, maintaining them in their undifferentiated state. By using this method it will be possible to study undifferentiated cancer stem cells *in vivo,* by injecting them in animal models.

The method according to the present invention was applied to epithelial cancers and it is based on the neurospheres model. Recent studies on neural stem cells demonstrated that an undifferentiated multi-potent population of neural cells is able to grow in suspension as neurospheres. Neurospheres contain from 4 to 20% of stem cells, the residual population being composed by progenitor cells in different phases of differentiation.

Starting from the neurosphere model and observing that normal epithelial cells can grow only if they are made to adhere to a matrix (conversely they undergo an apoptotic process, called anoikis), it has been found, according to the invention, that a small population of cancer cells with stem characteristic are able to survive and proliferate without adhering to a hexogenous support. These epithelial cancer cells were cultured without matrix, thus obtaining a cellular selection of a small number of cells that are able, to survive, proliferate and form multicellular spheres, compared to a majority of cells undergo anoikis.

Therefore, the present invention specifically provides a method to purify and amplify tumoral stem cells in undifferentiated state from solid tumours comprising the following steps:
a) obtaining tumour tissue portions from said solid tumours under sterile conditions;
b) dissociating said tumour tissue portions obtained from step a) by mechanical dispersion and/or enzymatic digestion;
c) washing and/or centrifuging the digest obtained from step b);
d) separating digest obtained from step c) in two aliquots, said two aliquots being washed and cultured with the respective culture medium (such as, e.g. DMEM plus FBS and DMEM/F12 plus hormone mix), with or without an adhesive substrate, to obtain, respectively, i) a control culture of primary cancer cells and ii) a culture of cancer stem cells in spheres;
e) centrifuging the culture ii) when sphere aggregates are formed and dissociating the said aggregates by mechanical dispersion and/or enzymatic digestion with a proteolytic enzyme, preferably collagenase (0,1 %);
f) subjecting the tumoral stem cells obtained from step e) to alternate cycles of static and shaking culture in a suitable culture medium.

Preferably, the solid tumour is an epithelial tumour. In particular, said epithelial tumour may chosen from the group consisting of thyroid, breast, prostate, bladder, colon, liver and lung cancer.

In a preferred embodiment of the method according to the invention, the enzymatic digestion of the tumour tissue portions according to step b) may be performed by incubation with at least an enzyme chosen from the group consisting of trypsin, collagenase, hyaluronidase, elastase, dispase and papain at a working concentration between 1000 U/ml to 3000 U/ml, at a temperature of 37°C and for an incubation time in the range of 60-300 minutes.

According to a preferred embodiment of the method of the invention the enzymatic digestion according to step b) is performed by incubating with collagenase, chosen from the group consisting of collagenase I, II and IV, at a working concentration between 30 U/ml and 1300 U/ml, at a temperature of 37°C and for an incubation time in the range of 60-300 minutes.

In the specific case of breast cancer tissue portions, the enzymatic digestion is performed by incubating with collagenase I at a working concentration of 500-1000 U/ml, at a temperature of 37°C and for an incubation time of 180-240 minutes.

Concerning the enzymatic digestion of prostate cancer tissue portions, the same is performed by incubating with collagenase II at a working concentration of 40-100 U/ml, preferably 40 U/ml, at a temperature of 37°C and for an incubation time of 100-130 minutes, preferably 120 minutes.

Concerning the enzymatic digestion of thyroid cancer tissue portions, the same is performed by incubating with collagenase II at a working concentration of 1250-2000 U/ml, preferably 1500 U/ml, at a temperature of 37°C and for an incubation time of 100-130 minutes, preferably 120 minutes.

In the case of treatment of bladder cancer tissue portions, the enzymatic digestion of the method according to the invention is performed by incubating with collagenase I at a working concentration of 1000-1500 U/ml, preferably 1250 U/ml, at a temperature of 37°C and incubated for 50-70 minutes, preferably 60 minutes.

In particular, the enzymatic digestion of colon cancer tissue portions is performed by incubating with collagenase I at a working concentration of 100-1500 U/ml, preferably 100 U/ml, at temperature of 37°C and for an incubation time of 90-210 minutes, preferably 180 minutes.

In the specific case of liver cancer tissue portions, the enzymatic digestion according to the method of the present invention is performed by incubating with collagenase IV at a working concentration of 250-1000 U/ml, preferably 500 U/ml, at a temperature of 37°C and for an incubation time of 20-90 minutes, preferably 60 minutes.

According to a particular aspect of the method described in the present invention, the culture medium of step d) to obtain i) a control culture of primary cancer cells may comprise the following components in solution:
DMEM 1x; FBS 10 ± 5 %; penicillin 50-150 U/ml; streptomicin 2-3 µg/ml; L-glutamine 1-4 mM; gentamicine 2-3 µg/ml; amphotericin 2-3 µg/ml.

The cells are cultured in normal flasks and when they come to confluence, they must be trypsinised and expanded or used for staining, and they will serve ad as control for cancer stem cells of the same tissue.

Similarly, the culture medium of step d) to obtain ii) a culture of cancer stem cells in spheres may comprise the following components in solution:
- Sterile water 75 ± 10%
- DMEM/F12 10-5x 10 ± 5%
- Glucose 30% 2 ± 1%
- Sodium bicarbonate 7.5% 1.5 ± 0.75%
- Hepes 1M 0.5 ±0.2%
- Glutamine 1 ± 0.5%
- Heparin (2 mg/ml) 0.2 ±0.1%
- Hormone mix10x 10 ± 5%,
   the said solution being further supplemented with:

- BSA 1.5-2.5 g/500 ml
- BFGF 8-12 ng/ml
- EGF 18-22 ng/ml.

Preferably, the culture medium of step d) to obtain ii) a culture of cancer stem cells in spheres comprises the following components in solution:
- Sterile water 75%
- DMEM/F12 10-5x 10%
- Glucose 30% 2%
- Sodium bicarbonate 7.5% 1.5%
- Hepes 1M 0.5%
- Glutamine 1 %
- Heparin (2 mg/ml) 0.2%
- Hormone mix10x 10%
the said solution being further supplemented with:
- BSA 2 g/500 ml
- BFGF 10 ng/ml
- EGF 20 ng/ml

The said hormone mix may comprise the following components in solution:
- Sterile water 80.5 ± 10%
- DMEM/F-12 10x 10 ± 5%
- Glucose 30% 2 ± 1 %
- NaHCO₃ 7.5% 1.5 ± 0.5%
- Hepes 1M 0.5 ± 0.2%
said solution being supplemented with:
- apotrasferrin 0.5 -1.5 mg/ml
- insulin 50-150 mg/400 ml
- putrescin 35-45 mg/400 ml
- selenium 3x10⁻³M 35-45 µl/400 ml
- progesterone 2x10⁻³M 35-45 µl/400 ml

Preferably, the hormone mix comprises the following components:
- Sterile water 80,5
- DMEM/F-12 10x 10
- Glucose 30% 2
- NaHCO₃ 7.5% 1.5
- Hepes 1M 0.5
said solution being supplemented with:
- apotrasferrin 1 mg/ml
- insulin 100 mg/400 ml
- putrescin 38.64 mg/400 ml
- selenium 3x10⁻³M 40 µl/400 ml
- progesterone 2x10⁻³M 40 µl/400 ml

In a particular embodiment of the method according to the present invention, the step e) of centrifugation of the ii) culture is performed after 7-10 days of culture.

According to a further aspect of the present invention, cancer stem cells obtained from step e) are cultured with an adequate medium in flasks (ultra low attachment flasks by Corning-Mascia Brunelli) for at least 7 days.

The present invention will be described below for mere illustrative, but not limitative, purposes with particular reference to the examples and the figures shown herein, where:
Figures 1-2 show the procedure of subcutaneous injection of a small number of cancer stem cells respectively from colon and from thyroid cancer into nude mice, obtaining after three months the genesis of a subcutaneous cancer having the same histological characteristics of the source cancer;
Figure 3 shows the analysis of IL-4 expression and their relative receptors in all analysed cancer stem cells (breast and colon).
Figure 4 shows the sensitization to death of breast cancer cells mediated by CD95, TRAIL and chemotherapy, obtained by using IL-4 neutralizing Abs.

### Example 1: Cancer tissue dissociation

Thyroid, breast, prostate, bladder, colon, liver and lung cancers were obtained from surgical removal of the tumour.

The tumours were digested in different manner based on the kind of tumour. The only common step is the tumour portion preparation: working under laminar flux in a sterile P3 room, small portions of cancer tissues were placed in culture medium in sterile containers and supplemented with antibiotics/antimycotics in ice overnight.

Then, working in sterile room, necrotic tissue, capsule and visible collagen were eliminated and the tissue portions were washed in culture medium or in PBS to eliminate most of the red cells.

To disaggregate tumour tissues so obtained two methods can be adopted, i.e. mechanic dissociation and enzymatic digestion.

In the first case, the procedure to follow is the same for all tumours, and consists in cutting tumour fragments into small portions of about 1-2 mm by sterile scissors. Cutting can be made easier by using sterile Petri dishes.

Regarding the enzymatic digestion, several proteolytic enzymes may be employed, such as trypsin, collagenase (i.e. collagenase I, II, IV), hyaluronidase, elastase, dispase and papain. Based on the kind of tumour tissue, an optimization of the enzyme choice or of a combination thereof, and of their working concentration is necessary to obtain a better dissociation of the tumoral cells.

### Enzymatic digestion

### Thyroid

Thyroid cancer tissues and normal thyroid tissues were washed a few times with PBS and then DMEM at room temperature. Tissues were placed in sterile tube and incubated with cold DMEM supplemented with penicillin 100 U/ml, streptomycin 100 µg/ml, amphotericin 2.5 µg/ml, L-glutamine 4 mM and incubated in ice overnight.

Thyroid tissue portions were cut by sterile scissors and the mass obtained was washed a few times with DMEM by sedimentation. The obtained thyroid tissues were placed in a sterile 50 ml tube and enzymatically digested. 10 ml of DMEM, 10 ml of HBSS, 1.5 mg/ml of collagenase II were used per each 4-5 g of tissue. Digestion is carried out under shaking at 37°C for 120 minutes. After this procedure the suspended cells are made of single cancer cells.

As concerns the culture of the cells so obtained; cultures of the primary differentiated thyroid cells were carried out in DMEM supplemented with penicillin 100 U/ml, streptomycin 100 µg/ml, amphotericin 2.5 µg/ml, L- glutamine 4 mM, glucose 2.7mg/ml and FBS 10% in order to increase the crypts adhesion; later, FBS 1% was used and the medium was changed every three days. When the cells reached sub-confluence, they were detached with trypsin/EDTA (0.025/0.02%).

### Colon

Colon cancer tissues and normal colon tissues were washed a few times with an isotonic NaCl solution at room temperature. Tissues were placed in sterile tube and incubated with cold PBS supplemented with penicillin 100 U/ml, streptomycin 100 µg/ml, gentamycin 2.5 µg/ml, amphotericin 2.5 µg/ml, L- glutamine 4 mM and glucose 0.2%, and incubated in ice overnight.

Colon tissues were washed many times with cold isotonic NaCl solution. After several washings normal colon epithelium was taken off by scratching with a sterile glass, while the cancer was left as such. Colon tissue portions so obtained were placed in a sterile 50 ml tube and enzymatically digested. 10 ml of DMEM, 10 ml of HBSS, 100 U/ml of collagenase I, L-glutamine 2 mM, 100 U/ml of penicillin, gentamycin 2.5 µg/ml and amphotericin 2.5 µg/ml were used per each 4-5 g of tissue.

The digestion was performed for 40-50 minutes at 37°C. After this procedure the suspended cells are made of crypts and single cancer cells.

To isolate the crypts, the suspension was centrifuged 5 times on a 2% D-sorbitol gradient for 5 minutes at 50 g. The isolated crypts, making up the pellet, were washed with HBSS for 3 minutes at 65 g (one crypt is made of 200-300 epithelial cells).

As concerns the culture of the cells so obtained, 25 cm² and 75 cm² flasks were coated with collagen I (respectively, 70 and 210 µl of collagen I), Cultures were carried out in DMEM supplemented with penicillin 100 U/ml, streptomycin 100 µg/ml, gentamycin 2.5 µg/ml, amphotericin 2.5 µg/ml, transferrin 5 µg/ml, insulin 10 µg/ml, 0.15 mM non-essential amino acids, hydrocortisone 1 µg/ml, EGF 30 ng/ml, L-glutamine 4 mM, glucose 2.7 mg/ml. For the first 24 hrs FBS 10% was used in order to increase the crypts adhesion; later, FBS 1% was used and the medium was changed every three days. When the cells reached sub-confluence, they were detached with trypsin/EDTA (0.025/0.02%).

### Breast

Breast tumour has to be treated as soon as possible after surgical removal. The fragment obtained has to be divided into small portions by a sterile lancet.

To obtain a single cells suspension, digestion is carried out with collagenase I in DMEM at the concentration of 0.5-1 mg/ml (500-1000 U/ml), and then incubated at 37°C for 3-4 hours. After incubation, the obtained digest was washed with DMEM. The cells so obtained can be divided in two aliquots to afford, respectively, cancer spheres and primary cancer cells.

To obtain primary cultures, the cells were cultured in a medium consisting of with DMEM/F12 1:1 supplemented with FBS 10%, insulin 5 µg/ml and EGF 10 ng/ml.

### Prostate

The cancer tissues were placed on a sterile Petri dishes and washed a few times with HBSS to eliminate the excess red cells. Necrotic tissues were cut off by a sterile lancet and was cut into small portions of about 1 mm³; it is important to avoid performing the enzymatic digestion in the event that the tumoral cells appear to disaggregate into small clumps as a result of a slight pressure.

The enzymatic digestion is carried out by adding collagenase II to the medium, at a concentration of 40 U/ml for 2 hours at 37°C. The digest was washed a few times and the pellet obtained was resuspended in HBSS with 10% FBS.

The cells so obtained were plated into flasks previously coated with collagen I.

To obtain the spheres it is necessary to add to add in the culture medium hydrocortisone (10 nM), insulin (5 µg/ml), trasferrin, estradiol and selenium.

### Bladder

Bladder cancer tissues and normal bladder tissues were washed with cold PBS and incubated overnight with HBSS supplemented with antibiotics. After mechanically disaggregating the fragments by means of sterile scissors, the tissues were digested in 40 ml HBSS in the absence of Ca²⁺/Mg²⁺ and supplemented with MgSO₄ 0.8mM, 20 mM HEPES, 0.1% BSA and 50 mg collagenase I (1.25 mg/ml = 1250 U/ml) for 1 hour at 37°C under shaking.

Single smooth muscle cells were recovered by recovering the digest supernatant which meanwhile sedimented.

Primary differentiated cells were cultured in DMEM plus penicillin 100 U/ml , streptomycin 100 µg/ml, amphotericin 2.5 µg/ml, L- glutamine 4 mM, glucose 2.7mg/ml and FBS 10%. When the cells reached at confluence, trypsin/EDTA (0,025/0,02%) was used to detach.

### Liver

Liver cancer tissues and normal liver tissues were maintained in HBSS containing 0.5 mg/ml (500 U/ml) of collagenase IV, 0.02 mg/ml DNAse, 2% FBS and 0.6% BSA and softly mechanically disaggregated into small fragments by means of a lancet.

The solution obtained was incubated for 20-40 minutes at 3.7°C and immediately after it was filtered through a 100 µm mesh to remove clumps and undissociated cells. Collagenase was eliminated by centrifugation at 30 g for 1 minute and the pellet containing hepatocytes was resuspended in HBSS supplemented with FBS 10%, penicillin/streptomycin (500 µg/ml), fungizone (2 µg/ml) and 1% glutamine.

In the various enzymatic digestions of solid tumours described above, (in particular for thyroid cancer and breast cancer tissues) it has also been found that the simultaneous employment of collagenase (at a working concentration ranging between 10 and 50 U/ml) and hyaluronidase (at a working concentration ranging between 30 and 2000 U/ml) optimized the digestion allowing to obtain a high number of tumour cells.

After enymatically digesting the tumour tissues, according to the procedures described above, the supernatant of the digest obtained was divided into two aliquots (one for the primary cells culture and one for the spheres). The aliquots underwent washing by centrifugation at 800 rpm with the respective culture medium, and were placed in culture in low attachment flasks (ultra low attachment flasks of Corning-Mascia Brunelli) by placing about 1000 cells per flask.

Culture mediums used in the method according to the present invention are respectively :
a) culture medium (500 ml) to obtain cancer stem cells in spheres having the following composition:
   - Sterile water 375 ml
   - DMEM/F12 10-5x 50 ml
   - Glucose 30% 10 ml
   - Sodium bicarbonate 7.5% 7.5 ml
   - Hepes 1M 2.5 ml
   - Glutamine 5 ml
   - Heparin (2 mg/ml) 1 ml
   - BSA 2 gr
   - BFGF 5 mg
   - EGF 10 mg
   - Hormone mix 10x 50 ml
   Concerning hormone mix 10x (400 ml), the preferred composition was as follows:
   - DMEM/F-12 10x 40 ml
   - Glucose 30% 8 ml
   - NaHCO₃ 7.5% 6 ml
   - Hepes 1M 2 ml
   - Sterile water 322 ml
   This solution has to be supplemented with:
   - apotrasferrin 400 mg
   - insulin 100 mg
   - putrescin 38,64 mg
   - selenium 3x10⁻³M 40 µl
   - progesterone 2x10⁻³M 40 µl
b) culture medium to obtain primary cancer stem cells having the following preferred composition: DMEM 1x; FBS 10%; penicillin 100 U/ml, streptomycin 2.5 µg/ml, L- glutamine 2 mM, gentamycin 2.5 µg/ml, amphotericin 2.5 µg/ml.

### Cancer stem cells culture

Cells are maintained in serum free culture medium (DMEM/F-12) supplemented with EGF, bFGF, heparin and hormone mix (see medium a).

Spheres, after 7-10 days of culture are recovered by centrifugation at ed at 800 rpm and mechanically or enzimatically (with trypsin) disaggregated.

Cell culture was controlled for some days, as cancer stem cells have the characteristic to exponentially grow. After one week of culture, spheres were incubated in a shaker inside a 37°C incubator.

After one or two weeks of shaking culture, flasks were statically incubated.

### Example 2: Death induction in cancer stem cells

Once cancer stem cells were obtained from the various tissues, it was observed by immuno-histochemistry methods that these cells expressed IL-4 and IL-10 and their respective receptors. Actually, treatment of cancer stem cells with neutralizing anti-IL-4 antibody increased the sensitization to death by treatment with chemotherapeutic drugs. To verify the tumorigenic features and the chemotherapeutic-induced cell death obtained by *in vitro* studies, an *in vivo* model of nude mice (mice without thymus) was used. 5000-500000 cancer stem cells were injected subcutaneously after dilution 1:1 with Matrigel (BD).

The experiments described herein are related in particular with cancer stem cells obtained from thyroid, breast and colon, but comparable results are expected using the other kinds of tumours.

After 70-90 days from injection of cancer stem cells, a small mass was visible (approximately 18 mm of diameter) which was made up by the same histotype of the original stem cells source.

After *in loco* treatment of the new-generated tumour with neutralizing anti-IL-4 and anti-IL-10 antibodies (20 µg/ml once a week for three weeks) an 80% reduction of the tumour volume was obtained, compared with the treatment with control IgG.

The treatment with antibodies neutralizing some cytokines (such as in this case IL-4 and IL-10), together with the conventional chemotherapy, would delete cancer stem cells, which are responsible for the minimal residual disease leading to metastasis.

### Example 3: Study of apoptotic features of cancer stem cells

In order to evaluate the apoptotic features of cancer stem cells there have been studied and identified the molecules which controlled proliferation and apoptosis of normal and cancer stem cells such as FLIP, Bcl-2 and Bcl-xl and it was evaluated the expression of some stem cells markers such as Wnt, beta-catenin, nestin, vimentin, p-63, which were more expressed in cancer than in normal stem cells.

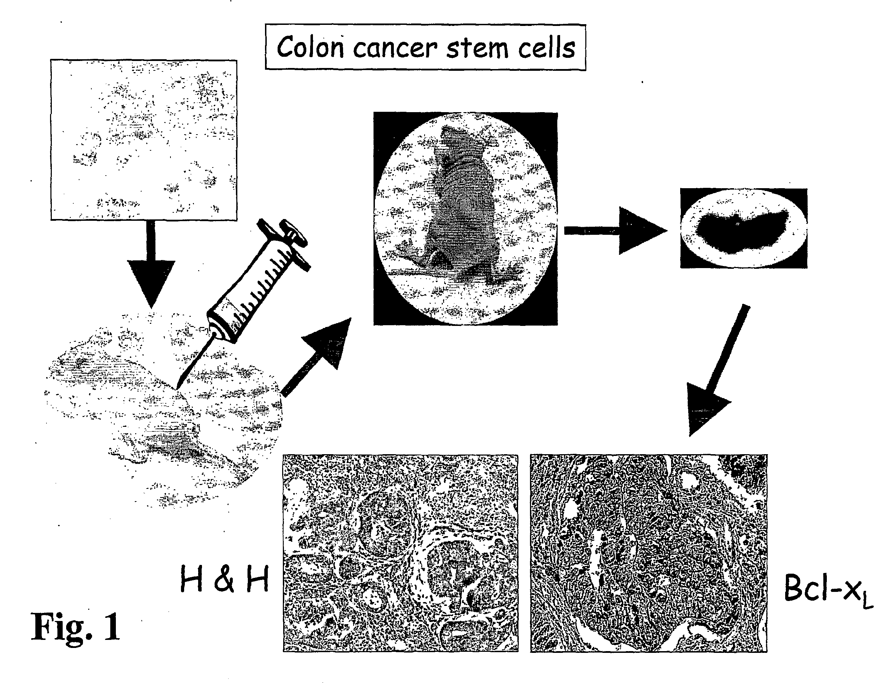

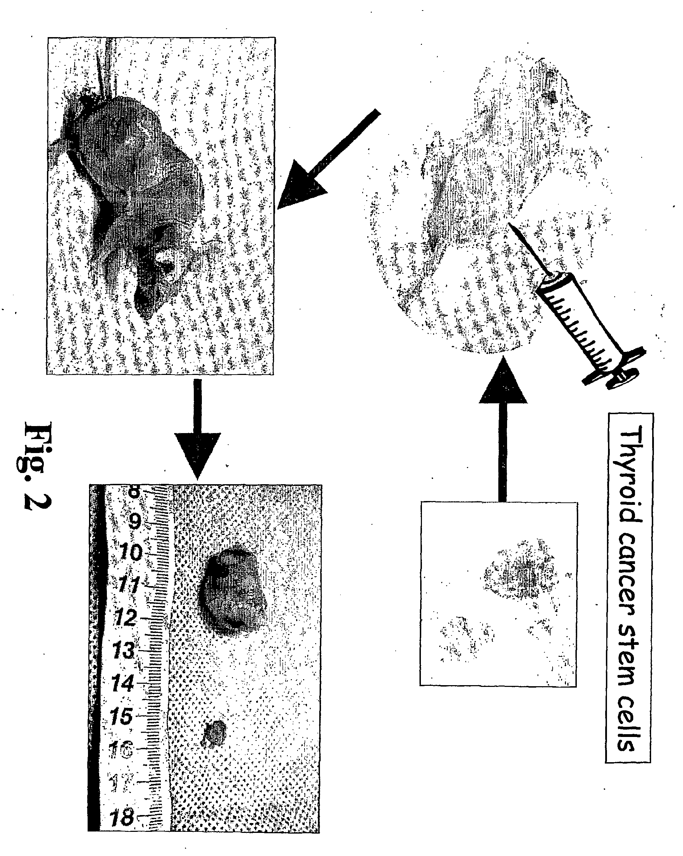

To confirm the tumorigenic features of isolated cancer stem cells, a small amount of cancer stem cells obtained from thyroid and from colon (500 cells) was injected subcutaneously in nude mice, obtaining after three months a subcutaneous tumour with the same histological features of original tumours (Fig. 1-2; respectively colon and breast cancer). The simultaneous injection in different mice of differentiated cancer cells did not give rise, as expected, to tumour mass formation.

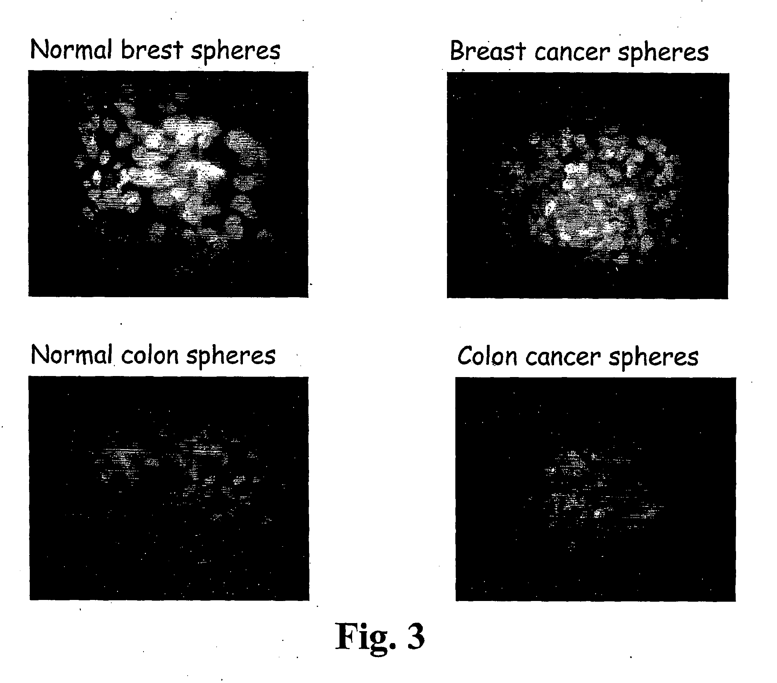

In has been observed that all cancer stem cells expose receptors and express high levels of IL-4 (Fig. 3).

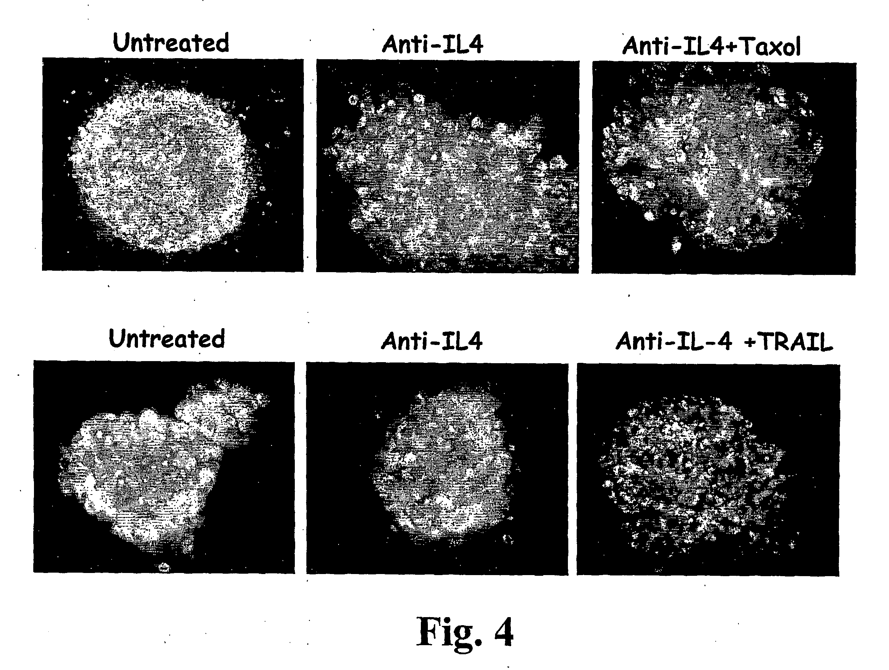

By inducing death through death receptors such as CD95 or TRAIL or by conventional chemotherapeutic drugs, the cancer stem cells were resistant to death. Only by pre-treating cancer stem cells with IL-4-neutralizing antibodies a massive sensitization to CD95 or TRAIL or chemotherapeutic drugs-induced cell death was obtained (Fig.4).

These cells represent the best biological source for the development of new therapeutic strategies which could lead to the complete eradication of the amount of neoplastic cells responsible of the maintenance and the development of a tumour. A contribution to the understanding of the rise and growth of a tumour derives from the study of the developmental biology of cancer stem cells.

The present invention has been disclosed with particular reference to some specific embodiments thereof, but it should be understood that modifications and changes may be made by the persons skilled in the art without departing from the scope of the invention as defined in the appended claims.

### REFERENCES

- Morrison, S. J., N. M. Shah, and D. J. Anderson. 1997. Cell 88:287.
- Weissman, I. L. 2000. Science 287:1442*.*
- Reya, T., S. J. Morrison, M. F. Clarke, and I. L. Weissman. 2001. Nature 414:105*.*
- Osawa, M., K. Hanada, H.' Hamada, and H. Nakauchi. 1996. Science 273:242*.*
- Morrison, S. J., and I. L. Weissman. 1994. Immunity 1:661*.*
- Baum, C. M., I. L. Weissman, A. S. Tsukamoto, A. M. Buckle, and B. Peault. 1992. Proc NatI Acad Sci U SA 89:2804*.*
- Spangrude, G. J., S. Heimfeld, and I. L. Weissman. 1988. Science 241:58*.*
- G. Stassi, M. Todaro, M. Zerilli, and R. De Maria. 2003. Cancer Research Oct. 15, 63(20): 6784-90.

### PREFERRED EMBODIMENTS OF THE INVENTION

1. A method for purifying and amplifying tumoral stem cells in undifferentiated state from solid tumours comprising the following steps:
   a) obtaining tumour tissue portions from said solid tumours under sterile conditions;
   b) dissociating said tumour tissue portions obtained from step a) by mechanical dispersion and/or enzymatic digestion;
   c) washing and/or centrifuging the digest obtained from step b);
   d) separating digest obtained from step c) in two aliquots, said two aliquots being washed and cultured with the respective culture medium, with or without an adhesive substrate, to obtain, respectively, i) a control culture of primary cancer cells and ii) a culture of cancer stem cells in spheres;
   e) centrifuging the culture ii) when sphere aggregates are formed and dissociating the said aggregates by mechanical dispersion and/or enzymatic digestion with a proteolytic enzyme;
   f) subjecting the tumoral stem cells obtained from step e) to alternate cycles of static and shaking culture in a suitable culture medium.
2. A method according to item 1, wherein said solid tumour is an epithelial tumour.
3. A method according to item 2, wherein said epithelial tumour is chosen from the group consisting of thyroid, breast, prostate, bladder, colon, liver and lung cancer.
4. A method according to any one of items 1-3, wherein the enzymatic digestion of the tumour tissue portions according to step b) is performed by incubation with at least an enzyme chosen from the group consisting of trypsin, collagenase, hyaluronidase, elastase, dispase and papain at a working concentration between 1000 U/ml to 3000 U/ml, at a temperature of 37°C and for an incubation time in the range of 60-300 minutes.
5. A method according to item 4, wherein the collagenase is chosen from the group consisting of collagenase I, II and IV, and the incubation is carried out at a working concentration between 30 U/ml and 1300 U/ml, at a temperature of 37°C and for an incubation time in the range of 60-300 minutes.
6. A method according to any one of items 1-5, wherein said enzymatic digestion of breast cancer tissue portions is performed by incubating with collagenase I at a working concentration of 500-1000 U/ml, at a temperature of 37°C and for an incubation time of 180-240 minutes.
7. A method according to any one of items 1-5, wherein said enzymatic digestion of prostate cancer tissue portions is performed by incubating with collagenase II at a working concentration of 40-100 U/ml, at a temperature of 37°C and for an incubation time of 100-130 minutes.
8. The method according to item 7, wherein the working concentration is 40 U/ml and the incubation time is 120 minutes.
9. A method according to any one of items 1-5, wherein said enzymatic digestion of thyroid cancer tissue portions is performed by incubating with collagenase II at a working concentration of 1250-2000 U/ml, at a temperature of 37°C and for an incubation time of 100-130 minutes.
10. The method according to item 9, wherein the working concentration is 1500 U/ml and the incubation time is 120 minutes.
11. A method according to any one of items 1-5, wherein said enzymatic digestion of bladder cancer tissue portions is performed by incubating with collagenase I at a working concentration of 1000-1250 U/ml, at a temperature of 37°C and for an incubation time of 50-70 minutes.
12. The method according to item 11, wherein the working concentration is 1250 U/ml and the incubation time is 60 minutes.
13. A method according to any one of items 1-5, wherein said enzymatic digestion of colon cancer tissue portions is performed by incubating with collagenase I at a working concentration of 100-1500 U/ml, at a temperature of 37°C and for an incubation time of 90-210 minutes.
14. The method according to item 13, wherein the working concentration is 100 U/ml and the incubation time is 180 minutes.
15. A method according to any one of items 1-5, wherein said enzymatic digestion of liver cancer tissue portions is performed by incubating with collagenase IV at a working concentration of 250-1000 U/ml, at a temperature of 37°C and for an incubation time of 20-90 minutes.
16. The method according to item 15, wherein the working concentration is 500 U/ml and the incubation time is 60 minutes.
17. A method according to any one of the preceding items wherein the culture medium of step d) to obtain i) a control culture of primary cancer cells may comprise the following components in solution:
   DMEM 1x; FBS 10 ± 5 %; penicillin 50-150 U/ml; streptomycin 2-3 µg/ml; L-glutamine 1-4 mM; gentamycin 2-3 µg/ml; amphotericin 2-3 µg/ml.
18. A method according to any one of the preceding items, wherein said culture medium of step d) to obtain ii) a culture of cancer stem cells in spheres comprises the following components in solution:
   - Sterile water 75 ±10%
   - DMEM/F12 10-5x 10 ± 5%
   - Glucose 30% 2 ± 1%
   - Sodium bicarbonate 7.5% 1.5 ± 0.75%
   - Hepes 1M 0.5 ± 0.2%
   - Glutamine 1 ± 0.5%
   - Heparin (2 mg/ml) 0.2 ± 0.1 %
   - Hormone mix10x 10 ± 5%,
   the said solution being further supplemented with:
   - BSA 1.5-2.5 g/500 ml
   - BFGF 8-12 ng/ml
   - EGF 18-22 ng/ml.
19. A method according to item 18, wherein said hormone mix comprises the following components in solution:
   - Sterile water 80.5 ± 10%
   - DMEM/F-12 10x 10 ± 5%
   - Glucose 30% 2 ± 1 %
   - NaHCO₃ 7.5% 1.5 ± 0.5%
   - Hepes 1M 0.5 ± 0.2%
   said solution being supplemented with:
   - apotrasferrin 0.5 - 1.5 mg/ml
   - insulin 50-150 mg/400 ml
   - putrescin 35-45 mg/400 ml
   - selenium 3x10⁻³M 35-45 µl/400 ml
   - progesterone 2x10⁻³M 35-45 µl/400 ml.
20. A method according to any one of the preceding items , wherein the step e) of centrifugation of the ii) culture is performed after 7-10 days of culture.
21. A method according to any one of the preceding items, wherein the proteolytic enzyme of step e) is collagenase or trypsin.
22. A method according to any one of the preceding items , wherein the cancer stem cells obtained from step e) are cultured with an adequate medium in flasks for at least 7 days.
23. A method according to any one of the preceding items , wherein comprising a further step g) of death induction in the tumour stem cells by means of cytokine-neutralizing agents.
24. A method according to item 23, wherein said neutralizing agents are antibodies.
25. A method according to items 23 or 24, wherein said cytokines are IL-4 and IL-10.
26. A culture medium for obtaining a culture of cancer stem cells in spheres comprising the following components in solution:
   - Sterile water 75 ±10%
   - DMEM/F12 10-5x 10 ± 5%
   - Glucose 30% 2 ± 1 %
   - Sodium bicarbonate 7.5% 1.5 ± 0.75%
   - Hepes 1M 0.5 ± 0.2%
   - Glutamine 1 ± 0.5%
   - Heparin (2 mg/ml) 0.2 ± 0.1%
   - Hormone mix10x 10 ± 5%,
   the said solution being further supplemented with:
   - BSA 1.5-2.5 g/500 ml
   - BFGF 8-12 ng/ml
   - EGF 18-22 ng/ml.
27. A culture medium according to item 24, wherein said hormone mix said hormone mix comprises the following components in solution:
   - Sterile water 80.5 ± 10%
   - DMEM/F-12 10x 10 ± 5%
   - Glucose 30% 2±1%
   - NaHCO₃ 7.5% 1.5 ±0.5%
   - Hepes 1M 0.5 ± 0.2%
   said solution being supplemented with:
   - apotrasferrin 0.5 - 1.5 mg/ml
   - insulin 50-150 mg/400 ml
   - putrescin 35-45 mg/400 ml
   - selenium 3x10⁻³M 35-45 µl/400 ml
   - progesterone 2x10⁻³M 35-45 µl/400 ml.

## Claims

1. Use of a cytokine-neutralizing agent for the manufacture of a medicament for inducing cancer stem cell death.

2. The use of claim 1, wherein the cytokines are IL-4 and/or IL-10.

3. The use of claim 1 or 2, wherein the cytokine-neutralizing agent is an antibody.

4. The use of any one of claims 1-3 for the treatment of minimal residual disease.

5. The use of any one of claims 1-4 for the treatment of metastasis.

6. The use of any one of claims 1-5, wherein the cancer stem cells are from a solid epithelial tumour, particularly chosen from the group consisting of thyroid, breast, prostate, bladder, colon, liver and lung cancer.

7. The use of any one of claims 1-6, wherein the medicament further comprises a chemotherapeutic drug.

8. The use of any one of claims 1-7, wherein the medicament further comprises an agent for inducing cell death through death receptors.
